# EUROPEAN PATENT SPECIFICATION

(11) **EP 2 453 759 B1**
(45) Date of publication and mention of the grant of the patent: **21.10.2015**
(21) Application number: 10734871.6
(22) Date of filing: 14.07.2010
(51) Int. Cl.: A23K 1/16, A23K 1/18, A61K 31/385, A61P 39/06

(54) **PET FOOD COMPOSITIONS INCLUDING A SUSTAINED-RELEASE LIPOIC ACID AND METHODS OF MANUFACTURE AND USE THEREOF**
HAUSTIERFUTTERMITTEL ENTHALTEND EINER VERZÖGERTEN FREISETZUNG FORM VON LIPONSÄURE, DEREN VERWENDUNG UND HERSTELLUNGSVERFAHREN
COMPOSITIONS D'ALIMENTS POUR ANIMAUX FAMILIERS COMPRENANT UNE FORME A LIBERATION PROLONGEE D'ACIDE LIPOIQUE, SON UTILISATION ET METHODE DE PRODUCTION

(30) Priority: 14.07.2009 US 225328 P
(43) Date of publication of application: 23.05.2012
(60) Divisional application: 15164858.1
(73) Proprietor: Hill's Pet Nutrition, Inc., Topeka, KS 66603 (US)
(72) Inventor: ZICKER, Steven C., Lawrence Kansas 66049 (US); GROSS, Kathy Lynn, Topeka Kansas 66618 (US)
(74) Representative: Daniels, Jeffrey Nicholas
(86) International application number: PCT/US2010/041888
(87) International publication number: WO 2011/008800

(56) References cited:
- WO-A1-2010/083409
- US-A1- 2003 044 466
- US-A1- 2004 166 157
- US-A1- 2009 176 864
- US-B1- 6 190 591
- US-B2- 6 572 888

## Description

### FIELD OF THE INVENTION

The invention encompasses pet food compositions that deliver a slow, sustained-release amount of lipoic acid or a salt thereof when fed orally to a companion animal. The invention also encompasses methods of manufacture of the compositions.

### BACKGROUND OF THE INVENTION

Lipoic acid has benefits to companion animals. For example, lipoic acid is important for growth and aging, helps to prevent cell damage, and helps the body rid itself of harmful substances. Natural occurring α-lipoic acid is found as lipoyl-lysine, R-alpha enantiomer, which is believed to undergo minimal cleavage prior to gastrointestinal absorption. In addition, de novo synthesis of α-lipoic acid from fatty acids and cysteine indicate that α-lipoic acid is synthesized in a protein bound form. Bioavailability of α-lipoic acid has been demonstrated to show enantiomeric differences in humans. The bioavailability of lipoyl-lysine has not been evaluated and may be different than free α-lipoic acid. It is well known that during processing of nutrients in commercial pet foods adducts of compounds may occur and impact bioavailability as seen with Maillard products and lysine. Administration of lipoic acid as a capsule with a meal has been evaluated and may impair absorption 10-20%. However, bioavailability of dl-alpha lipoic acid when added prior to extrusion of a pet food has not ever been evaluated and is not known.

### SUMMARY OF THE INVENTION

The inventors have developed pet food compositions having a slow-sustained release of lipoic acid when incorporated into a dry expanded pet food kibble. Compared to a single lipoic acid dose in capsule form, lipoic acid incorporated into pet food compositions of the invention results in a lower spike in blood levels of lipoic acid and sustains blood levels of lipoic acid over a longer period.

Accordingly, the inventors have developed pet food formulations including lipoic acid, which is an easy and convenient way for pet owners to dose the lipoic acid, so as to avoid large spikes in blood levels of lipoic acid that could be associated with side effects, and allows the lipoic acid to be delivered by sustained release to cells and tissues following the ingestion of the pet food formulation.

In one aspect, the present invention provides a pet food composition according to claim 1. Disclosed herein is a dried pet food composition having a food component wherein the food component comprises a slow, sustained-release amount of lipoic acid.

It has been found that lipoic acid remains bioavailable and can be delivered to pets over a slow sustained period of time when formulated into a coating on, distributed throughout, or filling in a dried pet food composition. Therefore, the invention offers the advantage of a ready-to-eat pet food composition, which is highly palatable and which contains a shelf stable source of lipoic acid delivered to a companion animal over a slow sustained release period to increase safety and efficacy of the lipoic acid administration.

In another aspect the invention provides a process according to claim 11. Disclosed is a process of preparing a dried, ready-to-eat pet food composition, the process including cooking a starch source to form a gelatinized starch matrix and forming the gelatinized starch matrix into pieces and drying the pieces, which contain lipoic acid such that the lipoic acid is delivered to pets over a slow, sustained period of time.

In another embodiment, the gelatinized starch matrix is formed into pieces and dried by extruding the gelatinized matrix to form a cooked extrudate and cutting and drying the cooked extrudate to form dried pieces, wherein the pieces include lipoic acid such that the lipoic acid is delivered to pets over a slow sustained period of time. The gelatinized matrix may be caused to expand upon extrusion to form, after cutting and drying, expanded pieces. In another embodiment, the gelatinized starch matrix may be formed into pieces and dried by roller drying the gelatinized starch matrix to form flakes.

In a further embodiment, the gelatinized starch matrix may be formed into pieces and dried by extruding the gelatinized matrix to form a cooked extrudate containing an aperture wherein the aperture includes a sustained-release material and lipoic acid such that the lipoic acid is delivered to pets over a slow sustained period of time; and cutting and drying the pieces.

In another embodiment, the invention encompasses a process of preparing a dried, ready-to-eat pet food composition, the process including cooking a starch source to form a gelatinized starch matrix; forming the gelatinized starch matrix into pieces and drying the pieces; and coating or filling the pieces with a substrate which contains lipoic acid such that the lipoic acid is delivered to pets over a slow sustained period of time.

Pet foods comprising lipoic acid are disclosed in e.g., US 2009/176864. Pet foods for the controlled release of actives are disclosed in e.g., US 2004/166 157.

### DETAILED DESCRIPTION OF THE INVENTION

The invention generally encompasses a pet food composition comprising a food component comprising (i) one or more pet food nutrient(s) and (ii) lipoic acid or a salt thereof, which allows a slow, sustained-release amount of lipoic acid. As used herein, the term "lipoic acid" includes lipoic acid and salts thereof. The pet food can be any form of pet food, which can include, but is not limited to, extruded, baked, rolled, crumbled, wet, or other pet food forms, and comprises a sustained-release material to allow the sustained release of lipoic acid. The sustained-release material includes hydroxypropyl methyl cellulose (HPMC).

In various embodiments, the sustained-release material is present in the compositions in amounts of about 4 to about 20 wt %, or about 6 to about 16 wt % or about 8 to about 12 wt %.

In certain embodiments, the lipoic acid is present in an amount of about 1 ppm to about 4500 ppm. In certain embodiments, the lipoic acid is present in an amount of about 10 ppm to about 2500 ppm. In certain embodiments, the lipoic acid is present in an amount of about 50 ppm to about 1500 ppm. In certain embodiments, the lipoic acid is present in an amount of about 100 ppm to about 1000 ppm. In certain embodiments, the lipoic acid is present in an amount of about 200 ppm.

In certain embodiments, the pet is a companion animal such as a dog or cat.

In certain embodiments, the food composition is in the form of a kibble.

In certain embodiments, the pet food composition is in the form of a pet snack.

In another embodiment, the invention encompasses a pet food kibble comprising:
(i) a gelatinized starch matrix; and
(ii) lipoic acid or a salt thereof; and
(iii) HPMC as the sustained-release material.

In certain embodiments, the lipoic acid is included in a coating or filling.

In certain embodiments, the gelatinized starch matrix is an extrusion cooked starch source.

In certain embodiments, the coating comprises a carrier substrate, which contains the lipoic acid.

In certain embodiments, the carrier substrate is at least one carrier chosen from the group consisting of a fat, a protein digest, milk solids, a sugar and a particulate flavoring agent.

In certain embodiments, the pet food kibble further includes a source of soluble fiber.

In another embodiment, the invention encompasses pet food compositions in the form of kibble, each kibble comprising:
(i) a gelatinized starch matrix which comprises a protein source; and
(ii) lipoic acid or a salt thereof; and
(iii) HPMC as the sustained-release material.

In certain embodiments, the lipoic acid is included in a coating or filling.

In certain embodiments, the coating comprises a carrier substrate, which contains the lipoic acid.

In certain embodiments, the carrier substrate is a fat, a protein digest, or a mixture thereof.

In certain embodiments, the kibble further includes a source of soluble fiber.

In another embodiment, the invention encompasses a process of preparing a pet food composition, the process comprising cooking a starch source and a protein source to form a gelatinized starch matrix containing protein; adding HPMC and lipoic acid; forming the gelatinized matrix into kibbles; and drying the kibbles.

In certain embodiments, the starch source and protein source are extrusion cooked; extruded through an orifice; and then cut into pieces, for example, kibbles.

In another embodiment, the invention encompasses a process of preparing a pet food composition, the process comprising cooking a starch source, a protein source, lipoic acid, and the sustained-release material, HPMC, to form a gelatinized starch matrix containing protein and lipoic acid; forming the gelatinized matrix into kibbles; and drying the kibbles.

In certain embodiments, the starch source and protein source are extrusion cooked; extruded through an orifice; and then cut into kibbles.

In another embodiment, the invention encompasses pet food kibble comprising:
(i) a gelatinized starch matrix which includes a component chosen from grains such as corn, rice, wheat, beets, barley, oats, soy, and combinations thereof; and
(ii) lipoic acid or a salt thereof and HPMC as sustained-release material.

In another embodiment, the invention relates to methods for promoting growth, preventing cell damage and/or helping rid the body of harmful substances in an animal in need thereof including administering to an animal a pet food composition comprising a food component, wherein the food component comprises a slow, sustained-release amount of lipoic acid to the animal.

As used herein, the term "nutrient" or "pet food nutrient" refers to a portion of the food composition, which can include up to about 100% of any particular food ingredient suitable for consumption by a companion animal or can include a mixture of food ingredients in various proportions. In certain embodiments, the pet food nutrient includes a combination of food ingredients in amounts of about 0 wt. % to about 50 wt. % fat, about 0 wt. % to about 75 wt. % carbohydrate, about 0 wt. % to about 95 wt. % protein, about 0 wt. % to about 40 wt. % dietary fiber, and about 0 wt. % to about 15 wt. % of one or more nutritional balancing agents.

The quantities administered in the diet, all as wt % (dry matter basis) of the diet, are calculated as the active material that is measured as free material.

The inventors have succeeded in developing a slow, sustained-release lipoic acid pet food composition. By using the composition of the invention in companion pets, it can be shown that an amount of lipoic acid can be delivered in a slow manner and can be maintained in the system for a longer period.

Lipoic acid or alpha-lipoic acid can be administered into the diet as alpha-lipoic acid or as a lipoate derivative as in U.S. Pat. No. 5,621,117, racemic mixtures, salts, esters or amides thereof. The quantity of alpha-lipoic acid can vary according to the age of the pet or the condition being treated. In certain embodiments, the amount of lipoic acid is about 25 ppm, about 50 ppm, about 100 ppm, about 200 ppm, about 500 ppm, about 1000 ppm, about 1500 ppm, about 2000 ppm, about 2500 ppm, about 3000 ppm, about 3500 ppm, about 4000 ppm, about 4500 ppm or about 5000 ppm. In various embodiments, the range of lipoic acid that can be administered to dogs is about 50 ppm to about 4500 ppm. In various embodiments, the range of lipoic acid that can be administered to cats is about 65 ppm to about 2600 ppm. Maximum quantities can vary from about 10 ppm to an amount which remains nontoxic to the pet.

In certain embodiments, the invention includes a dry pet food containing less than 15% moisture having a porous texture and appearance with fibrous food simulating pieces having a tough, pliable texture interspersed therein.

The pet food composition can also be in the form of snack products for companion animals.

In another embodiment, the pet food compositions of the invention may be produced from any suitable ingredients such as those commonly used in dried, ready-to-eat pet food products. One of these ingredients is a starch source. Suitable starch sources are, for example, grain flours such as corn, rice, wheat, barley, soy and oats. In addition, mixtures of these flours may be used. The flours may be whole flours or may be flours which have had fractions removed; for example, the germ fraction or husk fraction may be removed. Rice flour, corn flour and wheat flour are particularly suitable; either alone or in combination. The starch source will be chosen largely based on the nutritional value, palatability considerations, and the type of product desired.

In certain embodiments, the pet food composition may also contain a protein source. Suitable protein sources may be selected from any suitable animal or vegetable protein source; for example poultry meal, poultry by-product meal, chicken meal, chicken by-product meal, lamb meal, meat and bone meal, fish meal, soy bean meal, soy protein concentrates, milk proteins, corn gluten meal, wheat gluten, gluten and the like. The choice of the protein source will be largely determined by the nutritional needs, palatability considerations and the type of product desired. The starch source may also be a source of protein. In certain embodiments, the protein can be hydrolyzed or a protein isolate.

In various embodiments, the sustained-release material is present in the compositions in amounts of about 4 to about 20 wt %, or about 6 to about 16 wt % or about 8 to about 12 wt %.

In certain embodiments, the lipoic acid is incorporated into or onto a substrate, for example, a pet food kibble and a timed release or sustained-release coating is applied thereto. For example, the lipoic acid may be contained within or on a substrate as follows: (i) incorporated into matrix spheroids (e.g., together with an acceptable spheronizing agent such as cellulose), (ii) incorporated into a normal release core; or (iii) incorporated into a core which comprises a matrix including a sustained-release material. Thereafter, a sustained-release coating is applied onto substrates such as those mentioned in (i)-(iii) above. The pet food compositions including lipoic acid may optionally be coated with one or more materials suitable for the regulation of release or for the protection of the formulation. In one embodiment, coatings are provided to permit either pH-dependent or pH-independent release, for example, when exposed to gastrointestinal fluid.

In certain embodiments, the pet food compositions include a combination of one or more material(s) included with the pet food nutrients and lipoic acid, which is formed into a pellet or kibble, and which relies on diffusion or erosion to control release of the lipoic acid. For example, heterogeneous dispersions or solutions of lipoic acid and nutritional agents in water-swellable hydrogel matrices are useful in controlling the release of the lipoic acid by slow surface-to-center swelling of the matrix and subsequent release of the lipoic acid by a combination of diffusion of the nutrient and lipoic acid from the water-swollen part of the matrix and erosion of the water-swollen matrix containing the lipoic acid.

The pet food compositions include HPMC as sustained-release material, which provides for a sustained release of a lipoic acid according to a desired release profile through the use of one or more of the sustained-release ingredients described herein. In other embodiments, the sustained-release matrix system will provide a release profile, which releases lipoic acid at a substantially constant rate over a designated time period.

The controlled or sustained-release profile is obtained through the use of the sustained-release material, hydroxypropyl methyl cellulose (HPMC) as the primary ingredient of the sustained-release component. The sustained-release component can also contain amounts of other materials, which can affect the release profile. Examples of such materials include conventional waxes and waxy materials used in pharmaceutical formulations, such as carnuba wax, spermaceti wax, candellila wax, cocoa butter, cetosteryl alcohol, beeswax, partially hydrogenated vegetable oils, ceresin, paraffin, myristyl alcohol, stearyl alcohol, cetyl alcohol and stearic acid. Hydrophilic gums are also contemplated for use, in amounts, which can have an effect on the release profile. Examples of hydrophilic gums include acacia, gelatin, tragacanth, veegum, xanthan gum, carboxymethyl cellulose (CMC), hydroxy propyl cellulose (HPC) and hydroxy ethyl cellulose (HEC).

In various embodiments, the sustained-release material will contain about 4 to about 20 wt %, or about 6 to about 16 wt % or about 8 to about 12 wt % HPMC. The exact amount of the sustained-release material will vary depending upon the molecular weight of the substance and the desired release profile. For example, a pet food composition designed to provide a substantially constant release rate over a 12 hour period, which contains HPMC having a molecular weight of about 85,000, will contain about 8 to about 12 wt %, or about 10%, of the HPMC.

### Methods Of Making The Composition Of The Invention

The pet food composition fortified with lipoic acid may be produced in many different ways.

In one embodiment, the slow, sustained-release pet food compositions of the invention are made by an extrusion process, wherein the lipoic acid and sustained-release material including HPMC is incorporated in the pet food composition by co-manufacturing in the process of a complete extruded food meal.

The process involves blending a mixture to form a premix, cooking and extruding the mixture to form food pieces containing lipoic acid. The mixture is cooked and extruded to yield an expanded dry pet food composition having a porous texture and appearance containing food pieces having a tough, pliable fibrous texture. The respective proportions of the fibrous food pieces and basal matrix provide a food product, which has a uniform mixture that retains its particle integrity during production and upon storage.

The term "dry pet food" or "dry pet food material" is defined as one that has a moisture content less than 15% by weight and typically about 10%. All percentages referred to are understood to be by weight unless specified otherwise and are based upon the weight of the final product.

Conventionally, the dry pet food composition of the invention contains crude protein, crude fat, crude fiber, lipoic acid and other minerals and additives. Typical protein components include meat and bone meal and vegetable protein sources such as soybean meal. Other components are also suitable for use in this pet food product.

In still another variation, a portion of the lipoic acid is added with the other dry ingredients that are admixed with water, cooked and worked to form a partially fortified dough. Then, the balance of the lipoic acid can be admixed with the mixture to prepare a lipoic acid containing mixture fortified to desired levels.

The methods further comprise the step of forming the mixture into individual pieces of desirable shape and size (*e*.*g*., a kibble). Conventional techniques and equipment can be employed to practice this step and the skilled artisan will have no difficulty in selecting those suitable for use herein.

The dry pet food compositions can be fabricated into any of a variety of common forms including kibbles, biscuits, mini biscuits, flakes, or any snack product form, shape or size.

The methods further include the step of drying the shaped and sized individual pieces to form finished dry pet food compositions fortified with lipoic acid.

The skilled artisan will appreciate that the drying step depends in part upon the desired product.

In still another variation, the dry pet food composition containing lipoic acid can be extruded under conditions of temperature and pressure to puff and expand (the "direct expansion" technique) and sectioned or cut into individual pieces to form individual expansions. In this variation, the forming and drying steps are practiced simultaneously rather than sequentially.

In another variation, the drying step can involve heating the pieces under conditions that not only dry the piece but also cause the piece to expand to form dried and puffed or flaked finished pieces. For example, pellets can be gun puffed to form dried puffed pet food composition products.

The finished lipoic acid fortified dry pet food compositions, however formed, can optionally be provided with a topical coating and subsequently finish dried to remove the added moisture from the coating solution to form a finished dry pet food composition. In other variations, a topical coating optionally with salt and/or flavors is applied to form finished dried pet food compositions.

In commercial practice, one or more of the method steps can be combined and performed in or by a single piece of equipment. For example, a dry mix of ingredients including lipoic acid can be admixed with water and/or steam in a cooker extruder such as a single screw or twin screw. The cooker extruder heats, cooks and works the ingredients to form a lipoic acid containing dry pet food composition. In one variation, referred to in the art as direct expansion, the extruder conditions are such that upon extrusion, the cooked product expands and dries and is severed into small pieces. The pieces can be in final form. In slight variations, the pieces can be further dried to final moisture contents.

The dry pet food compositions so prepared can then be conventionally packaged for distribution and sale.

In another embodiment, the dry pet food compositions fortified with lipoic acid can be consumed by a companion animal to obtain the nutritional and physiological benefits of a high lipoic acid diet. A surprising advantage of the dry pet food composition is that the lipoic acid is nearly "invisible," that is, even high levels of lipoic acid are barely organoleptically discernible in the finished product. Surprisingly, the finished dry pet food composition products fortified with lipoic acid are similar to their unfortified counterparts, notwithstanding the presence of the added lipoic acid ingredient. The products are characterized by good flavor, good texture and other favorable organoleptic attributes. Accordingly, the invention also encompasses a pet food composition including a mixture of kibble some of which include a sustained-release amount of lipoic acid and other kibble which does not include lipoic acid.

To produce a pet food composition coated with lipoic acid, any technique suitable for coating the pieces may be used. For example, in the case of a liquid carrier substrate, the mixture of the lipoic acid and the carrier substrate may be sprayed onto the dried pieces. This may be carried out in any suitable manner. For example, the pieces may be fed into a fluidized bed onto which the mixture is sprayed. Alternatively, the pieces may be fed into a rotary coater into which the mixture is sprayed. As a further alternative, the pieces may be caused to fall in a curtain and the coating mixture sprayed onto the curtain. Heat sensitive components such as vitamins and amino acids may also be included in the dry mix. The dry mix is then agglomerated on the dried pieces using an agglomerating agent. Fats, oils and sugar solutions are examples of suitable agglomerating agents.

For a pet food composition product filled with lipoic acid, the mixture of the lipoic acid and carrier substrate is filled into the central bore of each piece. In this case, the carrier substrate is preferably viscous or a substance which hardens rapidly. Fats are particularly suitable. Alternatively, the pet food composition may be fed into a tumbler and the carrier substrate agglomerated to the product using a syrup. In this case, the product is coated and filled.

In order to illustrate without unduly limiting the novel aspects of the present invention, the following examples are presented.

### EXAMPLES

### Example 1 (not according to the invention)

A method for administration of lipoic acid to pets was investigated that would result in a slower, prolonged availability of lipoic acid to companion animals, for example, dogs and cats. Lipoic acid when administered as a capsule has a relatively rapid absorption and disappearance from serum. It is rapidly metabolized by the liver and excreted in the urine. The ability to have a method whereby the lipoic acid is made available over a longer time period but at lower levels may result in increased antioxidant efficacy and reduce potential toxicity to the animal. In addition, it is more convenient for a pet owner to provide the pet with a single food meal containing lipoic acid rather than trying dose capsules multiple times per day or risk harm to their pet by giving a single large close of lipoic acid.

A study is conducted to evaluate effect of dose and method of delivery on pharmacokinetics of orally administered dl-alpha-lipoic acid (ALA) in 27 healthy dogs. Three different doses of alpha-lipoic acid (2.5, 12.5 or 25 mg/kg bodyweight) and three variations of oral administration are evaluated: 1) lipoic acid capsule form administered after 12 hours of fasting, 2) lipoic acid capsule form given with a food meal (control food), 3) lipoic acid co-manufactured in the process of a complete extruded food meal. The control food without ALA is fed throughout the study period. The analytical results of the foods fed in this study are in Table 1.

**Table 1. Analytical results of foods fed on an as is basis.**

| Nutrient | Control | 2.5 mg/kg ALA | 12.5mg/kg ALA | 25 mg/kg ALA |
|---|---|---|---|---|
| Moisture (%) | 8.2 | 8.1 | 8.3 | 8.5 |
| Protein (%) | 23.5 | 23.9 | 23.3 | 23.9 |
| Fat (%) | 15.2 | 14.9 | 14.6 | 15.4 |
| Ash (%) | 4.9 | 5.0 | 4.8 | 5.1 |
| Calcium (%) | 0.74 | 0.81 | 0.74 | 0.81 |
| Phosphorus (%) | 0.54 | 0.58 | 0.56 | 0.59 |
| Lipoic Acid (ppm) | 15 | 191 | 602 | 1145 |

A three period Latin square design was used to create a 3x3 factorial arrangement of treatments with dogs assigned to a group following a planned treatment assignment. Serum is collected at 1 minute before alpha-lipoic acid dose and 15, 30, 45, 60, and 120 minutes after a single alpha lipoic acid dosing. Pharmacokinetic parameters are calculated using a non-compartmental model and analyzed using non-parametric tests of WinNonlin 4.1 in SAS v9.]. There was a significant effect of dose for all delivery methods (P<0.05). In addition, there was a significant effect of delivery method (p<0.05) with alpha lipoic acid incorporated in a single dry food meal resulting in lower maximum serum concentrations. Results of the study are in Table 2.

**Table 2: Mean ± Standard Deviation (n = number of animals in mean) for pharmacokinetic parameters (PK parameters) in dogs administered lipoic acid by different methodologies.**

| PK parameters | Administration rate 2.5 mg/kg | | | Administration rate 12.5 mg/kg | | | Administration rate 25 mg/kg | | |
|---|---|---|---|---|---|---|---|---|---|
| | SNF | SF | CMF | SNF | SF | CMF | SNF | SF | CMF |
| Cₘₐₓ (ng/ml) | 529 ± 724 (9) | 206 ± 91 (9) | 47 ± 12 (5) | 1696 ± 1847 (9) | 1151 ± 960(9) | 154 ± 59 (7) | 4037 ± 4171 (9) | 5441 ± 3777 (8) | 628 ± 994 (8) |
| AUC_{0-infininty} (min*ng/mL) | 18836 ± 19987 (6) | 8971 ± 1801 (7) | 33878 (1) | 74660 ± 44850 (8) | 59350 ± 19513 (8) | 92215 ± 25869(4) | 214531 ± 142814 (4) | 218029 ± 100463 (7) | 227304 ± 299998 (6) |
| Elimination half life (min) | 19.4 ± 9.7 (6) | 28.6 ± 22.6 (7) | 565 (1) | 29.1 ± 8.4 (8) | 45.1 ± 22.2 (8) | 379.2 ± 137.6 (4) | 46.7 ± 52.5 (4) | 30.2 ± 10 (7) | 573.3± 877.8 (6) |

| | | | | | | | | | |
|---|---|---|---|---|---|---|---|---|---|
| SNF= supplement provided as a capsule after fasting; SF=supplement as a capsule fed with food meal; CMF=Co-manufactured supplemental lipoic acid in an extruded dog food matrix. Cmax is the maximal concentration obtained in the plasma after administration of the lipoic acid at different doses. AUC0-infinity is the calculated area under the curve for lipoic acid as administered by the different routes and doses. Elimination half-life is the time to eliminate half the dose of lipoic acid from the plasma space. | | | | | | | | | |

As can be seen from Table 2, the maximal concentration of lipoic acid in plasma is significantly decreased, approximately 8-10 fold, when the substance is co-manufactured in the food thus reducing potential toxicity from higher plasma concentrations. In addition, the elimination half life and the total area under the curve are greater for the co-manufactured food which indicates that the lipoic acid co-manufactured with the food has a slower longer effective time of release to the plasma space.

## Claims

1. A pet food composition comprising a pet food nutrient, lipoic acid or a salt thereof, and a sustained-release material, wherein the sustained-release material comprises hydroxypropyl methyl cellulose (HPMC).

2. The pet food composition of claim 1, in the form of a kibble.

3. The pet food composition of claim 1, in the form of a pet snack.

4. The pet food composition of any preceding claim, further comprising a wax or waxy material suitable for use in a pharmaceutical formulation.

5. The pet food composition of claim 4, wherein the wax or waxy material is selected from: carnuba wax, spermaceti wax, candellila wax, cocoa butter, cetosteryl alcohol, beeswax, partially hydrogenated vegetable oils, ceresin, paraffin, myristyl alcohol, stearyl alcohol, cetyl alcohol and stearic acid.

6. The pet food composition of any preceding claim further comprising a hydrophilic gum.

7. The pet food composition of claim 6, wherein the hydrophilic gum is selected from: acacia, gelatin, tragacanth, veegum, xanthan gum, carboxymethyl cellulose, hydroxypropyl cellulose and hydroxyethyl cellulose.

8. The pet food composition of any preceding claim, wherein the HPMC is present in an amount from about 4 % to about 20 %, or about 6 % to about 16 %, or about 8 % to about 12 %.

9. The pet food composition of claim 8, wherein the HPMC has a molecular weight of about 85000 and is present in an amount from about 8 % to about 12 % or about 10 %, and the lipoic acid is released at a substantially constant rate over a 12 hour period.

10. The pet food composition of any preceding claim that is a cat food or a dog food.

11. A process of preparing a pet food composition, the process comprising:
cooking a starch source and a protein source to form a gelatinised starch matrix containing protein;
adding a sustained-release material and lipoic acid or a salt thereof;
forming the gelatinized matrix into kibbles;
and drying the kibbles,
wherein the sustained-release material is HPMC.

12. The process of claim 11, wherein the starch source and protein source are extrusion cooked; extruded through an orifice; and then cut into kibbles.

## Patentansprüche

1. Haustierfutterzusammensetzung, die einen Haustierfutternährstoff, Liponsäure oder ein Salz davon und ein Material mit anhaltender Freisetzung umfasst, wobei das Material mit anhaltender Freisetzung Hydroxypropylmethylcellulose (HPMC) umfasst.

2. Haustierfutterzusammensetzung nach Anspruch 1 in der Form einer Krokette.

3. Haustierfutterzusammensetzung nach Anspruch 1 in der Form eines Haustiersnacks.

4. Haustierfutterzusammensetzung nach einem vorhergehenden Anspruch, die weiterhin ein Wachs oder wachsartiges Material umfasst, das zur Verwendung in einer pharmazeutischen Formulierung geeignet ist.

5. Haustierfutterzusammensetzung nach Anspruch 4, wobei das Wachs oder wachsartige Material aus folgenden ausgewählt ist: Carnaubawachs, Walratwachs, Candelillawachs, Kakaobutter, Cetosterylalkohol, Bienenwachs, partiell hydrierte Pflanzenöle, Ceresin, Paraffin, Myristylalkohol, Stearylalkohol, Cetylalkohol und Stearinsäure.

6. Haustierfutterzusammensetzung nach einem vorhergehenden Anspruch, die weiterhin ein hydrophiles Gummi umfasst.

7. Haustierfutterzusammensetzung nach Anspruch 6, wobei das hydrophile Gummi aus folgenden ausgewählt ist:
Gummiarabikum, Gelatine, Tragantgummi, Veegum, Xanthan, Carboxymethylcellulose, Hydroxypropylcellulose und Hydroxyethylcellulose.

8. Haustierfutterzusammensetzung nach einem vorhergehenden Anspruch, wobei die HPMC in einer Menge von etwa 4 % bis etwa 20 % oder etwa 6 % bis etwa 16 % oder etwa 8 % bis etwa 12 % vorliegt.

9. Haustierfutterzusammensetzung nach Anspruch 8, wobei die HPMC ein Molekulargewicht von etwa 85.000 aufweist und in einer Menge von etwa 8 % bis etwa 12 % oder etwa 10 % vorliegt und die Liponsäure mit einer im Wesentlichen konstanten Rate über einen Zeitraum von 12 Stunden freigesetzt wird.

10. Haustierfutterzusammensetzung nach einem vorhergehenden Anspruch, bei der es sich um ein Katzenfutter oder ein Hundefutter handelt.

11. Verfahren zur Herstellung einer Haustierfutterzusammensetzung, wobei das Verfahren Folgendes umfasst:
Kochen einer Stärkequelle und einer Proteinquelle, um eine gelierte Stärkematrix zu bilden, die Protein enthält;
Zugeben von einem Material mit anhaltender Freisetzung und Liponsäure oder einem Salz davon;
Formen der gelierten Matrix zu Kroketten und Trocknen der Kroketten,
wobei das Material mit anhaltender Freisetzung HPMC ist.

12. Verfahren nach Anspruch 11, wobei die Stärkequelle und die Proteinquelle extrusionsgekocht; durch eine Düse extrudiert und dann zu Kroketten geschnitten werden.

## Revendications

1. Une composition alimentaire pour animaux de compagnie comprenant un nutriment alimentaire pour animaux de compagnie, de l'acide lipoïque ou ses sels et une substance à libération prolongée, dans laquelle la substance à libération prolongée comprend de l'hydroxypropyle méthyle cellulose (HPMC).

2. La composition alimentaire pour animaux de compagnie selon la revendication 1, sous la forme d'un croquette.

3. La composition alimentaire pour animaux de compagnie selon la revendication 1, sous la forme d'un snack pour animaux de compagnie.

4. La composition alimentaire pour animaux de compagnie selon l'une quelconque des revendications précédentes, comprenant en outre une cire ou une substance cireuse appropriée pour une utilisation dans une formulation pharmaceutique.

5. La composition alimentaire pour animaux de compagnie selon la revendication 4, dans laquelle la cire ou substance cireuse est sélectionnée parmi : cire de carnauba, cire de spermaceti, cire de candellila, beurre de cacao, alcool cétylstéarylique, cire d'abeille, huiles végétales partiellement hydrogénées, cérésine, paraffine, alcool myristylique, alcool stéarylique, alcool cétylique et acide stéarique.

6. La composition alimentaire pour animaux de compagnie selon l'une quelconque des revendications précédentes, comprenant en outre une gomme hydrophile.

7. La composition alimentaire pour animaux de compagnie selon la revendication 6, dans laquelle la substance de la gomme hydrophile est sélectionnée parmi : acacia, gélatine, tragacanthe, veegum, gomme de xanthane, carboxyméthyle cellulose, hydroxypropyle cellulose et hydroxyéthyle cellulose.

8. La composition alimentaire pour animaux de compagnie selon l'une quelconque des revendications précédentes, dans laquelle la HPMC est présente dans une quantité comprise entre environ 4 % et environ 20 %, ou entre environ 6 % et environ 16 %, ou entre environ 8 % et environ 12 %.

9. La composition alimentaire pour animaux de compagnie selon la revendication 8, dans laquelle la HPMC a un poids moléculaire d'environ 85 000 et est présente dans une quantité comprise entre environ 8 % et environ 12 % ou environ 10 %, et l'acide lipoïque est libéré à une cadence essentiellement constante pendant une période de 12 heure.

10. La composition alimentaire pour animaux de compagnie selon l'une quelconque des revendications précédentes, laquelle composition est un aliment pour chats ou pour chiens.

11. Un procédé de préparation d'une composition alimentaire pour animaux de compagnie, le procédé comprenant :
la cuisson d'une source d'amidon et d'une source de protéines pour former une matrice d'amidon gélatinisée contenant des protéines ;
l'ajout d'une substance à libération prolongée et d'acide lipoïque ou d'un de ses sels ;
la formation de la matrice gélatinisée en croquettes ;
et le séchage des croquettes,
dans laquelle la substance à libération prolongée est de la HPMC.

12. Le procédé selon la revendication 11, dans laquelle la source d'amidon et la source de protéines sont cuites par extrusion ; extrudées par un orifice ; puis coupées en croquettes.
